# EUROPÄISCHE PATENTSCHRIFT

(11) **EP 2 793 820 B1**
(45) Veröffentlichungstag und Bekanntmachung des Hinweises auf die Patenterteilung: **31.10.2018**
(21) Anmeldenummer: 12809239.2
(22) Anmeldetag: 17.12.2012
(51) Int. Cl.: A61K 8/36, A61K 8/40, A61Q 5/00

(54) **VERWENDUNG EINER WIRKSTOFFKOMBINATION AUS SORBINSÄURE UND EINER HYDROXAMSÄURE ZUR BEKÄMPFUNG VON KOPFSCHUPPEN.**
USE OF AN ACTIVE AGENT COMBINATION OF SORBIC ACID AND A HYDROXAMIC ACID AGAINST DANDRUFF
UTILISATION D'UNE ASSOCIATION D'ACIDE SORBIQUE ET D'UN ACIDE HYDROXAMIQUE POUR COMBATTRE LES PELLICULES CAPPILLAIRES.

(30) Priorität: 19.12.2011 DE 102011088962
(43) Veröffentlichungstag der Anmeldung: 29.10.2014
(73) Patentinhaber: Beiersdorf AG, 20253 Hamburg (DE)
(72) Erfinder: FIRYN, Andreas, 21493 Schwarzenbek (DE); GÖKSEL, Hülya, 50733 Köln (DE); KÖHLER, Manuela, 22147 Hamburg (DE); KOHUT, Michaela, 20257 Hamburg (DE); PILZNER, Anke, 22459 Hamburg (DE); TRAUPE, Bernd, 24568 Kaltenkirchen (DE)
(86) Internationale Anmeldenummer: PCT/EP2012/075706
(87) Internationale Veröffentlichungsnummer: WO 2013/092457

(56) Entgegenhaltungen:
- WO-A1-99/59568
- WO-A1-2006/029896
- WO-A1-2009/070736
- WO-A2-2006/029818
- DE-A1- 2 060 764
- DE-A1- 19 922 538
- US-A- 4 661 342
- Anonymous: "Staphylococcus aureus", , 18 May 2017 (2017-05-18), XP055374294, Retrieved from the Internet: URL:https://de.wikipedia.org/wiki/Staphylo coccus_aureus [retrieved on 2017-05-18]
- Anonymous: "Kopfschuppen", , 18 May 2017 (2017-05-18), XP055374299, Retrieved from the Internet: URL:https://de.wikipedia.org/wiki/Kopfschu ppen [retrieved on 2017-05-18]

## Beschreibung

Die vorliegende Erfindung betrifft die nichttherapeutische Verwendung einer Wirkstoffkombination umfassend Sorbinsäure und eine oder mehrere physiologisch unbedenkliche Hydroxamsäuren zur Bekämpfung oder Prophylaxe von Kopfschuppen.

Die Beschreibung zeigt auch Wirkstoffkombinationen und Zubereitungen, solche Wirkstoffkombinationen enthaltend, sowie deren Verwendung als gegen Bakterien, Mycota und Viren wirksame Substanzen.

Der gesunde warmblütige Organismus, insbesondere die gesunde menschliche Haut, ist mit einer Vielzahl nichtpathogener Mikroorganismen besiedelt. Diese sogenannte Mikroflora der Haut ist nicht nur unschädlich, sie stellt einen wichtigen Schutz zur Abwehr opportunistischer oder pathogener Keime dar.

Bakterien gehören zu den prokaryotischen Einzellern. Sie können grob nach ihrer Form (Kugel, Zylinder, gekrümmter Zylinder) sowie nach dem Aufbau ihrer Zellwand (grampositiv, gramnegativ) unterschieden werden. Feinere Unterteilungen tragen auch der Physiologie der Organismen Rechnung. So existieren aerobe, anaerobe sowie fakultativ anaerobe Bakterien. Manche Individuen sind in ihrer Eigenschaft als pathogene Keime von medizinischer Bedeutung, andere wiederum sind vollkommen harmlos.

Gegen Bakterien wirksame Substanzen sind seit geraumer Zeit bekannt. Der Begriff "Antibiotika" beispielsweise, der nicht auf alle antimikrobiell wirksamen Substanzen anwendbar ist, läßt sich auf das Jahr 1941 datieren, obwohl die ersten Erkenntnisse zum Penicillin bereits im Jahre 1929 gefunden wurden. Antibiotika im heutigen Sinne sind nicht für alle medizinischen, schon gar nicht kosmetische Anwendungen geeignet, da häufig auch der warmblütige Organismus, also etwa der erkrankte Patient, bei Anwendung auf irgendeine Weise in seinen Stoffwechselfunktionen beeinträchtigt wird.

Gramnegative Keime sind beispielsweise Escherichia coli, Pseudomonas-Arten sowie Enterobacteriaceen, wie etwa Citrobacter.

Auch grampositive Keime spielen in Kosmetik und Dermatologie eine Rolle. Bei der unreinen Haut beispielsweise sind neben anderen Einflüssen bakterielle Sekundärinfektionen von ätiologischer Bedeutung. Einer der wichtigsten Mikroorganismen, der in Zusammenhang mit unreiner Haut steht, ist Propionibacterium acnes.

Unreine Haut und/oder Komedonen beeinträchtigen das Wohlbefinden der Betroffenen aber selbst in leichten Fällen. Da praktisch jeder oder jede Jugendliche von unreiner Haut irgendeiner Ausprägung betroffen ist, besteht bei vielen Personen Bedarf, diesem Zustande abzuhelfen.

Kosmetische Desodorantien sind Formulierungen die dazu dienen, Körpergeruch zu beseitigen, der entsteht, wenn der an sich geruchlose frische Schweiß durch insbesondere grampositive Mikroorganismen zersetzt wird. Den üblichen kosmetischen Desodorantien liegen unterschiedliche Wirkprinzipien zugrunde.

Bekannt und gebräuchlich sind sowohl flüssige Desodorantien, beispielsweise Aerosolsprays, Roll-ons und dergleichen als auch feste Zubereitungen, beispielsweise Deo-Stifte ("Sticks"), Puder, Pudersprays, Intimreinigungsmittel usw.

In sogenannten Antitranspirantien kann durch Adstringentien - vorwiegend Aluminiumsalze wie Aluminiumhydroxychlorid (Aluchlorhydrat) - die Entstehung des Schweißes unterbunden werden. Abgesehen von der Denaturierung der Hautproteine greifen die dafür verwendeten Stoffe aber, abhängig von ihrer Dosierung, drastisch in den Wärmehaushalt der Achselregion ein und sollten allenfalls in Ausnahmefällen angewandt werden.

Durch die Verwendung antimikrobieller Stoffe in kosmetischen Desodorantien kann die Bakterienflora auf der Haut reduziert werden. Dabei sollten im Idealfalle nur die Geruch verursachenden Mikroorganismen wirksam reduziert werden. In der Praxis hat sich aber herausgestellt, dass die gesamte Mikroflora der Haut beeinträchtigt werden kann.

Der Schweißfluß selbst wird dadurch nicht beeinflußt, im Idealfalle wird nur die mikrobielle Zersetzung des Schweißes zeitweilig gestoppt.

Auch die Kombination von Adstringentien mit antimikrobiell wirksamen Stoffen in ein und derselben Zusammensetzung ist gebräuchlich. Die Nachteile beider Wirkstoffklassen lassen sich auf diesem Wege jedoch nicht vollständig beseitigen.

Schließlich kann Körpergeruch auch durch Duftstoffe überdeckt werden, eine Methode, die am wenigsten den ästhetischen Bedürfnissen des Verbrauchers gerecht wird, da die Mischung aus Körpergeruch und Parfümduft eher unangenehm riecht.

Allerdings werden die meisten kosmetischen Desodorantien, wie auch die meisten Kosmetika insgesamt, parfümiert, selbst wenn sie desodorierende Wirkstoffe beinhalten. Parfümierung kann auch dazu dienen, die Verbraucherakzeptanz eines kosmetischen Produktes zu erhöhen oder einem Produkt ein bestimmtes Flair zu geben.

Die Parfümierung wirkstoffhaltiger kosmetischer Mittel, insbesondere kosmetischer Desodorantien, ist allerdings nicht selten problematisch, weil Wirkstoffe und Parfümbestandteile gelegentlich miteinander reagieren und einander unwirksam machen können.

Desodorantien sollen folgende Bedingungen erfüllen:
1) Sie sollen eine zuverlässige Desodorierung bewirken.
2) Die natürlichen biologischen Vorgänge der Haut dürfen nicht durch die Desodorantien beeinträchtigt werden.
3) Die Desodorantien müssen bei Überdosierung oder sonstiger nicht bestimmungsgemäßer Anwendung unschädlich sein.
4) Sie sollen sich nach wiederholter Anwendung nicht auf der Haut anreichern.
5) Sie sollen sich gut in übliche kosmetische Formulierungen einarbeiten lassen.

Pilze, auch Fungi [fungus = lat. Pilz], Mycota [µυκης = grch. Pilz] oder Mycobionten genannt, zählen im Gegensatze zu den Bakterien zu den Eucaryonten. Eucaryonten sind Lebewesen, deren Zellen (Eucyten) im Gegensatz zu denen der sogenannten Procaryonten (Procyten) über einen durch Kernhülle und Kernmembran vom restlichen Cytoplasma abgegrenzten Zellkern verfügen. Der Zellkern enthält die Erbinformation in Chromosomen gespeichert.

Zu Vertretern der Mycobionten zählen beispielsweise Hefen (Protoascomycetes), Schimmelpilze (Plectomycetes), Mehltau (Pyrenomycetes), der falsche Mehltau (Phycomycetes) und die Ständerpilze (Basidiomycetes).

Pilze, auch nicht die Basidiomyceten, sind keine pflanzlichen Organismen, haben aber wie diese eine Zellwand, zellsaftgefüllte Vakuolen und eine mikroskopisch gut sichtbare Plasmaströmung. Sie enthalten keine photosynthetischen Pigmente und sind C-heterotroph. Sie wachsen unter aeroben Bedingungen und gewinnen Energie durch Oxidation organischer Substanzen. Einige Vertreter, beispielsweise Hefen, sind allerdings fakultative Anaerobier und zur Energiegewinnung durch Gärungsprozesse befähigt.

Dermatomycosen sind Krankheiten, bei der gewisse Pilzarten, insbesondere Dermatophyten, in die Haut und Haarfollikel eindringen. Die Symptome von Dermatomycosen sind beispielsweise Bläschen, Exfoliation, Rhagaden und Erosion, meist verbunden mit Juckreiz oder allergischem Ekzem.

Dermatomycosen können im wesentlichen in folgende vier Gruppen unterteilt werden: Dermatophyten (z.B. Epidermophytie, Favus, Mikrosporie, Trichophytie), Hefemycosen (z.B. Pityriasis und andere Pityrosporum-bedingte Mykosen, Candida-Infektionen, Blastomycose, Busse-Buschke-Krankheit, Torulose, Piedra alba, Torulopsidose, Trichosporose), Schimmelmycosen (z.B. Aspergillose, Kephalosporidose, Phycomycose und Skopulariopsidose), Systemmycosen (z.B. Chromomycose, Coccidiomycose, Histoplasmose).

Zu den pathogenen und fakultativ pathogenen Keimen gehören beispielsweise aus der Gruppe der Hefen Candida-Arten (z.B. Candida albicans) und solche der Familie Pityrosporum. Pityrosporum-Arten, insbesondere Pityrosporum ovale, sind für Hauterkrankungen wie Pityriasis versicolor, Seborrhoe in den Formen Seborrhoea oleosa und Seborrhoea sicca, welche sich vor allem als Seborrhoea capitis (= Kopfschuppen) äußern, seborrhoisches Ekzem und Pityrosporum-Follikulitis verantwortlich zu machen. Eine Beteiligung von Pityrosporum ovale an der Entstehung von Psoriasis wird von der Fachwelt diskutiert.

Alle Bereiche der menschlichen Haut können von Dermatomycosen befallen werden. Dermatophyten befallen fast ausschließlich Haut, Haare und Nägel. Hefemycosen können auch Schleimhäute und innere Organe befallen, Systemmycosen erstrecken sich regelmäßig auf ganze Organsysteme.

Besonders häufig sind die Körperbereiche betroffen, auf welchen sich durch Kleidung, Schmuck oder Schuhwerk Feuchtigkeit und Wärme stauen können. So gehört der Fußpilz zu den bekanntesten und am weitesten verbreiteten Dermatomycosen. Besonders unangenehm sind weiterhin Pilzerkrankungen der Finger- und Fußnägelbereiche (Onychomykosen).

Ein weit verbreitetes Phänomen sind die sogenannten Kopfschuppen. Produzieren die Talgdrüsen eines Menschen zu viel Talg oder sind diese Talgdrüsen durch zu seltenes Haare waschen durch Talg verstopft, dann wird dem normalerweise harmlose Hefepilz Pitysporum ovale, heute bekannt als Malassezia Furfur, ein starkes Wachstum ermöglicht, was zum Seborrhöischen Ekzem mit Juckreiz und Schuppenbildung führt.

Eine Aufgabe der vorliegenden Erfindung war, die Nachteile des Standes der Technik zu beseitigen und Substanzen und Zubereitungen, solche Substanzen enthaltend, zur Verfügung zu stellen, durch deren Verwendung die Bildung von Kopfschuppen zu unterbinden, wobei die physiologische Hautflora keine nennenswerte Einbußen erleidet.

Es is an sich bekannt, dass Superinfektionen der Haut durch Pilze und Bakterien nicht selten sind.

Bei bestehendem Primärinfekt, d.h., der normalen Keimbesiedelung der Haut, eintretende Neuinfektion mit hohen Keimzahlen eines oder mehrerer oft physiologischer Erreger, beispielsweise Staphylokokken, oft aber auch unphysiologischer Erreger, beispielsweise Candida albicans, kann bei Zusammentreffen ungünstiger Einflüssen eine "Superinfektion" der befallenen Haut auftreten. Die normale Mikroflora der Haut (oder eines anderen Körperorgans) wird dabei von dem Sekundärerreger regelrecht überwuchert.

Solche Superinfektionen können sich, in Abhängigkeit vom betreffenden Keim, in günstig verlaufenden Fällen in unangenehmen Hauterscheinungen (Juckreiz, unschönes äußeres Erscheinungsbild) äußern. In ungünstig verlaufenden Fällen können sie aber zu großflächiger Zerstörung der Haut führen, im schlimmsten Falle sogar im Tode des Patienten gipfeln.

Superinfektionen der vorab geschilderten Art sind z.B. beim Vollbild von AIDS häufig auftretende Sekundärerkrankungen. An sich - jedenfalls in geringen Keimdichten -unschädliche, aber unter Umständen auch ausgesprochen pathogene Keime überwuchern auf diese Weise die gesunde Hautflora. Bei AIDS allerdings sind auch andere Körperorgane von Superinfektionen betroffen.

Ebenso werden derartige Superinfektionen bei einer Vielzahl dermatologischer Erkrankungen, z.B. atopischem Ekzem, Neurodermitis, Akne, seborrhoischer Dermatitis oder Psoriasis beobachtet. Auch viele medizinische und therapeutische Maßnahmen, z.B die Radio- oder Chemotherapie von Tumorerkrankungen, als Nebenwirkung hervorgerufene, medikamentös induzierte Immunsuppression oder aber systemische Antibiotikabehandlung, ebenso wie externe chemische oder physikalische Einflüsse (z.B. Umweltverschmutzung, Smog), fördern das Auftreten von Superinfektionen der äußeren und inneren Organe, insbesondere der Haut und der Schleimhäute.

Zwar ist es im Einzelfalle ohne weiteres möglich, Superinfektionen mit Antibiotika zu bekämpfen, meistens haben solche Substanzen aber den Nachteil unangenehmer Nebenwirkungen. Oft sind Patienten beispielsweise gegen Penicilline allergisch, weswegen eine entsprechende Behandlung sich in einem solchen Falle verbieten würde.

Ferner haben topisch verabreichte Antibiotika den Nachteil, dass sie die Hautflora nicht nur vom Sekundärerreger befreien, sondern auch die an sich physiologische Hautflora stark beeinträchtigen und der natürliche Heilungsprozeß auf diese Weise wieder gebremst wird.

Aufgabe der vorliegenden Erfindung war, die Nachteile des Standes der Technik zu beseitigen und Substanzen und Zubereitungen, solche Substanzen enthaltend, zur Verfügung zu stellen, durch deren Verwendung Superinfektionen geheilt werden können, wobei die physiologische Hautflora keine nennenswerte Einbußen erleidet.

Protozoen sind parasitisch lebende Einzeller mit klar abgegrenztem Zellkern, die sich ungeschlechtlich fortpflanzen (durch Zwei- oder Vierfachteilung sowie Knospung), oder aber geschlechtlich (Gameto-, Gamonto- und Autogamie). Die Nahrungsaufnahme aus der Umgebung erfolgt durch Permeation sowie durch Pino- oder Phagozytose. Die meisten Protozoen können neben vegetativen, meist beweglichen Zustandsformen (sogenannten Trophozoiten) unter ungünstigen Umständen auch Zysten als Dauerformen ausbilden,

Je nach Fortbewegungsart und -apparat werden Protozoen in vier verschiedene Gruppen unterteilt:
(a) Mastigophora (Flagellaten mit Geißeln)
(b) Sarcodina/Rhizopoda (amöboides Bewegungsmuster durch Plasmaausstülpungen)
(c) Sporozoa (schlängelndes oder gleitendes Bewegungsmuster)
(d) Ciliata/Ciliophora (Bewimperung oder Begeißelung)

Parasitisch lebende Protozoen werden in subtropischen und tropischen Gebieten häufig durch stechende und saugende Insekten, aber auch Schmutz- und Schmierinfektion sowie durch die Nahrungskette übertragen.

Einige medizinisch und dermatologisch relevante Protozoonosen sind: Trichomoniasis (verursacht von Trichomonas vaginalis), Lamblienruhr (verursacht durch Lamblia intestinalis), viszerale sowie kutane und Schleimhaut-Leishmaniose (verursacht beispielsweise durch Leishmania donovanii, L.tropica, L.brasiliensis, L-mexicana, L.diffusa oder L. pifanoi), Trypanosmiasis (verursacht durch verschiedene Trypanosoma-Arten), Amöbenruhr und Amöbiasis (verursacht beispielsweise durch verschiedene Entamoeba-Arten, Jodamoeba butschlii oder Naegleria fowleri), Kokzidose (durch Isospora belli) und Balantidenruhr (verursacht durch Balantidium coli).

Durch Protozoonosen hervorgerufene medizinische und dermatologische Phänomene beeinträchtigen, zum Teil erheblich, das menschliche Wohlbefinden. Es besteht daher bei den betroffenen Personen ein erheblicher Bedarf, diesem Zustande abzuhelfen. Eine Aufgabe der vorliegenden Erfindung war es also, gegen Protozoen wirksame Wirkprinzipien zu finden.

Im Gegensatze zu den prokaryotischen und eukaryotischen zellulären Organismen sind Viren [Virus = lat. Gift] biologische Strukturen, welche zur Biosynthese eine Wirtszelle benötigen. Extrazelluläre Viren (auch "Virionen" genannt) bestehen aus einer ein- oder doppelsträngigen Nukleinsäuresequenz (DNS oder RNS) und einem Proteinmantel (Capsid genannt), gegebenenfalls einer zusätzlichen lipidhaltigen Hülle (Envelope) umgeben. Die Gesamtheit aus Nukleinsäure und Capsid wird auch Nucleocapsid genannt. Die Klassifikation der Viren erfolgte klassisch nach klinischen Kriterien, heutzutage allerdings zumeist nach ihrer Struktur, ihrer Morphologie, insbesondere aber nach der Nukleinsäuresequenz.

Medizinisch wichtige Virengattungen sind beispielsweise Influenzaviren (Familie der Orthomyxoviridae), Lyssaviren (z.B. Tollwut, Familie der Rhabdoviren) Enteroviren (z.B. Hepatitis-A, Familie der Picornaviridae), Hepadnaviren (z.B. Hepatitis-B, Familie der Hepadnaviridae).

Viruzide, also Viren abtötende Substanzen im eigentlichen Sinne gibt es nicht, da Viren nicht über eigenen Stoffwechsel verfügen. Es wurde aus diesem Grunde auch diskutiert, ob Viren als Lebewesen eingeordnet werden sollten. Pharmakologische Eingriffe ohne Schädigung der nicht befallenen Zellen sind jedenfalls schwierig. Mögliche Wirkmechanismen im Kampfe gegen die Viren sind in erster Linie die Störung deren Replikation, z.B. durch Blockieren der für die Replikation wichtigen Enzyme, die in der Wirtszelle vorliegen. Ferner kann das Freisetzen der viralen Nukleinsäuren in die Wirtszelle verhindert werden. Im Rahmen der hiermit vorgelegten Offenbarung wird unter Begriffen wie "antiviral" oder "gegen Viren wirksam", "viruzid" oder ähnlichen die Eigenschaft einer Substanz verstanden, einen ein- oder mehrzelligen Organismus vor schädlichen Folgen einer Virusinfektion, sei es prophylaktisch oder therapeutisch, zu schützen, ungeachtet dessen, was der tatsächliche Wirkmechanismus der Substanz im Einzelfalle sei.

Es ist letztendlich auch notwendig, kosmetische Zubereitungen langzeitstabil gegen mikrobielle Kontamination zu formulieren.

Die mikrobielle Stabilität wird bislang durch den Zusatz an Konservierungsmitteln gelöst. Bekannte Konservierungsmittel sind zum Beispiel die als Parabene bezeichneten Verbindungen, insbesondere 4-Hydroxybenzoesäure und deren Ester, Methylparaben, Ethylparaben, Propylparaben, Isopropylparaben, Butylparaben, Isobutylparaben, Phenylparaben.

Zubereitungen ohne oder möglichst geringer Menge an Konservierungsmittel bereit zu stellen ist jedoch ein Wunsch der Konsumenten.

Es wurde gefunden, dass die Verwendung einer Wirkstoffkombination umfassend Sorbinsäure und eine oder mehrere physiologisch unbedenkliche Hydroxamsäuren zur Bekämpfung oder Prophylaxe von Kopfschuppen verwendet werden kann.

Hydroxamsäuren sind eine Klasse chemischer Verbindungen, die als funktionelle Gruppe die Gruppierung -CO-NHOH enthalten. Ein Beispiel ist die Octanohydroxamsäure, auch Caprylohydroxamsäure genannt, welche durch folgende Struktur gekennzeichnet ist.

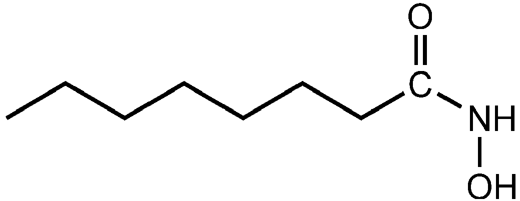

Sie ist ein bekannter Wirkstoff zur Konservierung kosmetischer Zubereitungen (z.B. WO 2009/070736 A1)

Sorbinsäure ist durch folgende Struktur gekennzeichnet:

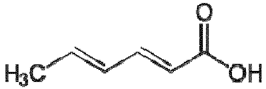

Es ist erfindungsgemäß vorteilhaft, die Gewichtsverhältnisse von Hydroxamsäure, insbesondere Octanohydroxamsäure, einerseits zu der Gesamtmenge an einer oder mehreren Carbonsäuren andererseits aus dem Bereich von 1 zu 10 bis 10 zu 1, bevorzugt von 1 zu 5 bis 5 zu 1, insbesondere bevorzugt von 1 zu 2 bis 2 zu 1 zu wählen.

Vorteilhaft werden die erfindungsgemäßen Wirkstoffkombinationen in kosmetischen oder dermatologischen Zubereitungen eingesetzt.

Erfindungsgemäße Zubereitungen enthalten vorteilhaft 0,001 bis 5,0 Gew.-% Hydroxamsäure, insbesondere Octanohydroxamsäure, bevorzugt 0,01 bis 2,0 Gew.-%, bezogen auf das Gesamtgewicht der Zubereitungen.

Erfindungsgemäße Zubereitungen enthalten vorteilhaft 0,001 bis 5,0 Gew.-% an Sorbinsäure, bevorzugt 0,01 bis 2,0 Gew.-%, jeweils bezogen auf das Gesamtgewicht der Zubereitungen.

Vorteilhaft werden die Gewichtsverhältnisse der Hydroxamsäure, insbesondere Octanohydroxamsäure, einerseits zu der Gesamtmenge an Sorbinsäure rerseits, aus dem Bereich 50 : 1 bis 1 : 50, vorzugsweise 5 : 1 bis 1 : 5 gewählt.

Die mit dieser Wirkstoffkombination hergestellten Zubereitungen stellen in jeglicher Hinsicht überaus befriedigende Präparate dar.

Diese kosmetischen und/oder dermatologischen Formulierungen können wie üblich zusammengesetzt sein und zur Behandlung der Haut und/oder der Haare im Sinne einer dermatologischen Behandlung oder einer Behandlung im Sinne der pflegenden Kosmetik dienen. Sie können aber auch in Schminkprodukten in der dekorativen Kosmetik eingesetzt werden.

Es hat sich in erstaunlicher Weise herausgestellt, dass die beschriebenen Wirkstoffkombinationen das Wachstum von Staphylococcus. Aureus verhindern, und dies in synergistischer Weise, also überadditiv in Bezug auf die Einzelkomponenten.

Es hat sich in erstaunlicher Weise herausgestellt, daß die beschriebenen verwendeten Wirkstoffe das Wachstum von grampositiven und gramnegativen Bakterien, Mycobionten verhindert.

Die beschriebenen Wirkstoffe eignen sich darüber hinaus gut für die Verwendung als desodorierender Wirkstoff in kosmetischen Desodorantien sowie gegen unreine Haut, leichte Formen der Akne bzw. Propionibakterium acnes.

Die beschriebenen Wirkstoffe haben sich ebenfalls als besonders wirkungsvoll gegen Streptokokken erwiesen.

Schließlich hat sich herausgestellt, daß die beschriebenen Wirkstoffe den Verderb organischer Substanz, insbesondere kosmetischer und dermatologischer Zubereitungen, durch den Befall mit grampositiven und gramnegativen Bakterien, Mycobionten und Viren verhindern können, wenn sie diesen Zubereitungen zugesetzt werden

Die beschriebenen Wirkstoffkombinationen sind ausgezeichnet wirksam gegen Mycobionten, insbesondere in deren Auswirkungsform der Dermatomycosen. Dabei sind die erfindungsgemäßen Wirkstoffkombinationen insbesondere befähigt, das Wachstum von Hefen, insbesondere der Pityrosporum-Arten, namentlich Pityrosporum ovale, zu verhindern.

Es hat sich ferner in überraschender Weise herausgestellt, dass die erfindungsgemäßen Wirkstoffkombinationen die Bildung von seborrhoischen Erscheinungen, insbesondere Kopfschuppen, verhindern sowie bereits vorhandene seborrhoische Erscheinungen, insbesondere Kopfschuppen, zu beseitigen.

Erfindungsgemäß ist somit die Verwendung einer Wirkstoffkombination umfassend Sorbinsäure und eine oder mehrere physiologisch unbedenkliche Hydroxamsäuren zur Bekämpfung oder Prophylaxe von Kopfschuppen.

Es hat sich auch herausgestellt, dass die beschriebenen Wirkstoffkombinationen den Verderb organischer Substanz, insbesondere kosmetischer und dermatologischer Zubereitungen, durch den Befall mit Mycobionten verhindern können, wenn sie diesen Zubereitungen zugesetzt werden.

Ferner war erstaunlich, dass die erfindungsgemäßen Wirkstoffkombinationen besonders gut wirksam sind gegen den für das Entstehen von Kopfschuppen verantwortlichen Keim Pityrosporum ovale und verwandte Keime. In einer besonderen Ausführungsform der vorliegenden Erfindung, wird die Wirkstoffkombination in gegen Kopfschuppen anzuwendende Formulierungen eingearbeitet, beispielsweise in Antischuppenshampoos.

Zur Anwendung werden diese kosmetischen und/oder dermatologischen Formulierungen in der für Kosmetika und Dermatika üblichen Weise auf die Haut und/oder die Haare in ausreichender Menge aufgebracht.

Vorteilhaft sind solche kosmetische und dermatologische Zubereitungen, die in der Form eines Sonnenschutzmittels vorliegen. Vorteilhaft enthalten diese zusätzlich mindestens einen UVA-Filter, UVB und/oder mindestens einen Breitbandfilter und/oder mindestens ein anorganisches Pigment.

Kosmetische Zubereitungen gemäß der Erfindung zum Schutze der Haut vor UV-Strahlen können in verschiedenen Formen vorliegen, wie sie z.B. üblicherweise für diesen Typ von Zubereitungen eingesetzt werden. So können sie z.B. eine Lösung, eine Emulsion vom Typ Wasser-in-ÖI (W/O) oder vom Typ Öl-in-Wasser (O/W), oder eine multiple Emulsionen, beispielsweise vom Typ Wasser-in-ÖI-in-Wasser (W/O/W), ein Gel, eine Hydrodispersion, einen festen Stift oder auch ein Aerosol darstellen.

Die kosmetischen Zubereitungen können kosmetische Hilfsstoffe enthalten, wie sie üblicherweise in solchen Zubereitungen verwendet werden, z.B. Konservierungsmittel, Bakterizide, Antioxidantien, Parfüme, Mittel zum Verhindern des Schäumens, Farbstoffe, Pigmente, die eine färbende Wirkung haben, Verdickungsmittel, oberflächenaktive Substanzen, Emulgatoren, weichmachende Substanzen, anfeuchtende und/oder feuchhaltende Substanzen, Fette, Öle, Wachse oder andere übliche Bestandteile einer kosmetischen Formulierung wie Alkohole, Polyole, Polymere, Schaumstabilisatoren, Elektrolyte, organische Lösungsmittel, Puder oder Silikonderivate.

Emulsionen sind gemäß der Erfindung vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Gele enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Feste Stifte enthalten z.B. natürliche oder synthetische Wachse, Fettalkohole oder Fettsäureester. Bevorzugt werden Lippenpflegestifte sowie desodorierende Stifte ("Deo-Sticks").

Als Treibmittel für aus Aerosolbehältern versprühbare kosmetische oder dermatologische Zubereitungen sind die üblichen bekannten leichtflüchtigen, verflüssigten Treibmittel, z.B. Kohlenwasserstoffe (Propan, Butan, Isobutan) geeignet, die allein oder in Mischung miteinander eingesetzt werden können. Auch Druckluft ist vorteilhaft zu verwenden.

Vorteilhaft können die Zubereitungen zudem Substanzen enthalten, die UV-Strahlung im UVB-Bereich absorbieren, wobei die Gesamtmenge der Filtersubstanzen z.B. 0,1 Gew.-% bis 40 Gew.-%, vorzugsweise 0,5 bis 10 Gew.-%, insbesondere 1 bis 6 Gew.-% beträgt, bezogen auf das Gesamtgewicht der Zubereitung, um kosmetische Zubereitungen zur Verfügung zu stellen, die die Haut vor dem gesamten Bereich der ultravioletten Strahlung schützen.

Bei kosmetischen Zubereitungen zur Pflege der Haare handelt es sich beispielsweise um Shampoonierungsmittel, Zubereitungen, die beim Spülen der Haare vor oder nach der Shampoonierung, vor oder nach der Dauerwellbehandlung, vor oder nach der Färbung oder Entfärbung der Haare angewendet werden, um Zubereitungen zum Fönen oder Einlegen der Haare, Zubereitungen zum Färben oder Entfärben, um eine Frisier- und Behandlungslotion, einen Haarlack oder um Dauerwellmittel.

Die kosmetischen Zubereitungen enthalten Wirkstoffe und Hilfsstoffe, wie sie üblicherweise für diesen Typ von Zubereitungen zur Haarpflege und Haarbehandlung verwendet werden.

Als Hilfsstoffe dienen Konservierungsmittel, oberflächenaktive Substanzen, Substanzen zum Verhindern des Schäumens, Emulgatoren, Verdickungsmittel, Fette, Öle, Wachse, organische Lösungsmittel, Bakterizide, Parfüme, Farbstoffe oder Pigmente, deren Aufgabe es ist, die Haare oder die Zubereitung selbst zu färben, Elektrolyte, Zubereitungen gegen das Fetten der Haare.

Kosmetische Zubereitungen, die ein Shampoonierungsmittel oder eine Wasch-, Dusch- oder Badezubereitung darstellen, enthalten vorzugsweise mindestens eine anionische, nicht-ionische oder amphotere oberflächenaktive Substanz oder Gemische daraus, Wirkstoffkombinationen gemäß der Erfindung, und Hilfsmittel, wie sie üblicherweise dafür verwendet werden.

Die folgenden Beispiele sollen die Verkörperungen der vorliegenden Erfindungen verdeutlichen, wobei nur die Formulierungen 3, 4, 7, 8 und 14 für die erfindungsgemäße Verwendung geeignet sind. Die Angaben beziehen sich stets auf Gewichts-%, sofern nicht andere Angaben gemacht werden.

### O/W Emulsion:

| Beispiel Nr. | **1** | **2** | **3** | **4** | **5** |
|---|---|---|---|---|---|
| Panthenol | 1,5 | 0 | 0 | 0,7 | 0 |
| Octanohydroxamsäure | 0,25 | 0,2 | 0,3 | 0,1 | 0,2 |
| Benzoesäure | 0,2 | 0,25 | 0 | 0 | 0 |
| Sorbinsäure | 0 | 0 | 0,2 | 0,15 | 0 |
| Paraffinum Liquidum | 0 | 3 | 3 | 0 | 0 |
| Lävulinsäure | 0 | 0 | 0 | 0 | 0,3 |
| Isopropyl Palmitat | 1 | 0 | 0 | 0 | 0 |
| Caprylic/Capric Triglycerid | 0 | 3 | 3 | 0 | 1,00 |
| Cetearylalkohol | 0 | 0 | 0 | 2,5 | 4,00 |
| Cetylalkohol | 0 | 3 | 3 | 0 | 0 |
| Paraffinum Liquidum | 0 | 3 | 3 | 0 | 0 |
| Dimethicon | 0 | 3 | 3 | 0 | 0,50 |
| Cyclomethicon D5 | 0 | 3 | 3 | 0 | 0 |
| C12-15 Alkylbenzoat | 0 | 0 | 0 | 0 | 2,00 |
| Butyrospermum Parkii Butter | 0 | 0,5 | 0,5 | 0 | 0 |
| Dicaprylylether | 0 | 0 | 0 | 10 | 0 |
| Dicaprylylcarbonat | 2 | 0 | 0 | 0 | 0 |
| Glyceryl Stearate | 0 | 1,2 | 1,2 | 2,4 | 2,4 |
| Tapiocastärke aq. | 1 | 0 | 0 | 0 | 0 |
| Glycerin | 5 | 8 | 10 | 8 | 8 |
| Butyleneqlykol | 0 | 0 | 0 | 1 | 0 |
| Zitronensäure | 0,0050 | 0 | 0 | 0 | 0 |
| Natronlauge aq 45% | 0,35 | 0,01 | 0,01 | 0,25 | 1 |
| Polyglyceryl-10 Myristat | 0 | 1,8 | 0 | 0 | 0 |
| Polyglyceryl-10 Stearat | 0,7 | 0 | 1,8 | 1 | 1 |
| Carbopol 981 | 0,5 | 0,02 | 0,02 | 0 | 0 |
| Acrylic Acid/VP Crosspolymer | 0 | 0 | 0 | 0,5 | 0,75 |
| Trinatrium EDTA aq. | 0 | 1 | 1 | | 1 |
| Ethylhexylmethoxycinnamat + BHT | 0 | 0 | 0 | 2 | 0 |
| Butyl Methoxydibenzoylmethane | 0 | 0 | 0 | 0 | 2 |
| Phenylbenzimidazolesulfonsäure | 0 | 0 | 0 | 0 | 2 |
| Ethylhexylsalicylat | 0 | 0 | 0 | 0 | 2 |
| Titandioxid + Trimethoxycaprylylsilan | 0 | 0 | 0 | 0,3600 | 0 |
| Octocrvlen | 0 | 0 | 0 | 0 | 2 |
| Parfum | 0,10 | 0,35 | 0,35 | 0 | 0,20 |
| Wasser | ad 100 | ad 100 | ad 100 | ad 100 | ad 100 |

### Hydrodispersionsgele

| **Beispiel Nr.** | **6** | **7** | **8** | **9** | **10** |
|---|---|---|---|---|---|
| Silikonöl, cyclisch | 8 | 10 | 0 | 0 | 0 |
| Silikonöl, linear | 0 | 0 | 0 | 0 | 3 |
| Dimethiconol | 1 | 2 | 3 | 0 | 3 |
| Ethanol | 1,0 | 0 | 7,5 | 0 | 3,0 |
| Natriumpolyacrylat | 0,2 | 0,3 | 0,3 | 0,4 | 0,10 |
| Methylpropandiol | 0 | 0 | 4 | 0 | 0 |
| Glycerin | 9 | 15 | 5 | 7,5 | 25 |
| Carbomer | 0,2 | 0,3 | 0,2 | 0,4 | 0,15 |
| Acrylate/C10-30Alkylacrylat Crosspolymer | 0,2 | 0,15 | 0,3 | 0,4 | 0,10 |
| Carrageenan (Chondrus Crispus) | 0 | 0 | 0 | 0 | 2 |
| Hamamelis Extrakt | 0,1 | 0,2 | 0,3 | 0,4 | 0 |
| Parfum | q.s. | q.s. | q.s. | q.s. | q.s. |
| Octanohydroxamsäure | 0,25 | 0,2 | 0,3 | 0,1 | 0,2 |
| Benzoesäure | 0 | 0 | 0 | 0,5 | 0,1 |
| Sorbinsäure | 0 | 0,5 | 0,4 | 0 | 0 |
| Milchsäure | 1 | 0 | 0 | 0 | 0 |
| Wasser | ad 100 | ad 100 | ad 100 | ad 100 | ad 100 |

### W/O-Emulsionen

| | **11** | **12** | **13** | **14** | **15** |
|---|---|---|---|---|---|
| Triglycerindiisostearat | 1,0 | 0,5 | 0,25 | 2,0 | 3,0 |
| Diglycerindipolyhydroxystearat | 1,0 | 1,5 | 1,75 | 3,0 | 2,0 |
| Paraffinöl | 12,5 | 10,0 | 8,0 | 5,0 | 11,5 |
| Vaseline | 8,0 | 6,0 | 5,0 | 12,0 | 2,5 |
| hydrierte Kokosglyceride | 2,0 | 1,0 | 2,5 | 5,0 | 0,25 |
| Decyloleat | 0,5 | 0,75 | 1,0 | 2,0 | 0,25 |
| Octyldodecanol | 0 | 0 | 0 | 0 | 0,25 |
| Aluminiumstearat | 0,4 | 0,3 | 0,6 | 1,0 | 0,05 |
| Dicaprylylcarbonat | 0,1 | 0,05 | 0 | 0 | 1,0 |
| Hydriertes Rizinusöl | 0 | 0,75 | 1,0 | 2,5 | 5,0 |
| Chitosan | 0,5 | 0 | 0 | 0 | 0 |
| Polyacrylsäure, Na-Salz | 0 | 0 | 0 | 0,15 | 0,25 |
| Magnesiumchlorid | 0,5 | 0,6 | 0,5 | 0,7 | 1,0 |
| Glycerin | 3,0 | 5,0 | 10,0 | 15,0 | 1,5 |
| Milchsäure | 0,5 | 0,9 | 0,2 | 0 | 0 |
| Parfum | q,s, | q,s, | q,s, | q,s, | q,s, |
| Ethanol | 2,0 | --- | 5,0 | --- | --- |
| Capryl-/Caprinsäuretriglycerid | 2,0 | 2,5 | 3,0 | 5,0 | 0,5 |
| Octanohydroxamsäure | 0,5 | 0,5 | 1,0 | 0,25 | 0,25 |
| Benzoesäure | 0 | 0,5 | 0,1 | 0 | 0 |
| Salicylsäure | 0,1 | 0 | 0 | 0 | 0 |
| Sorbinsäure | 0 | 0 | 0 | 0,25 | 0 |
| Wasser | ad 100 | ad 100 | ad 100 | ad 100 | ad 100 |

### Wirknachweis:

Das Bakterium *S.aureus (ATCC 12000)* wurde aus einem nach EN 12353 angelegten Cryoröhrchen auf einer entsprechenden Agarplatte (TSA-Agar) ausgestrichen und bei 37°C 24 Stunden bebrütet. Von den gewachsenen Bakterien wurde eine zweite Passage angelegt, die in der Wachstumskurve eingesetzt wurde.

Eine Impföse der Bakterien wurde zu 10mL Verdünnungsmittel und 5g Glasperlen mit 4mm Durchmesser gegeben und 3 Minuten gevortext. Anschließend wurde diese Bakteriensuspension auf eine OD von 0,08 eingestellt, um eine Keimzahl von ungefähr 5*10⁷ zu gewährleisten. Diese Bakteriensuspension wurde anschließend für den Test eingesetzt.

Die Wachstumskurve wurde mittels TECAN-Pipettierroboter durchgeführt. Hierzu wurden 500µL der zu testenden Wirkstofflösungen und 500 µl der Bakteriensuspension in Eppendorfgefäßen ohne Deckel mit aufgeschweißter Folie im Cooling-Rack vorgelegt. Nach 1, 6 und 24 Stunden bei 20°C wurden aus den Eppendorfgefäßen jeweils 100µL entnommen und zu 800µL Neutralisationsmittel und 100µl Wasser pipettiert (10⁻¹-Verdünnung). Aus der 10⁻¹-Verdünnung wurde eine 10⁻³-Verdünnung hergestellt (990µl Verdünnungsmittel und 10µl aus der 10⁻¹-Verdünnung). Diese Verdünnungen wurden mittels Spiralplatter auf Agarplatten ausplattiert.

Eine Ausnahme stellt die Kontrolle da, die nur zu Beginn abgenommen wurde. Sie wurde nochmal 1:100 verdünnt (10⁻⁵-Verdünnung). Bei den Kontrollen wurden die 10⁻³ und 10⁻⁵ - Verdünnungen auf Agarplatten gebracht. Zum Schluss wurden die Platten bei 37°C 24 Stunden bebrütet und anschließend mit Hilfe des Countermaten ausgewertet.

In der Auswertung wurde der Reduktionsfaktor bestimmt. Der Reduktionsfaktor ist der Faktor, um den die Zellzahl (CFU) der Testsubstanz im Vergleich zur Kontrolle reduziert wird.

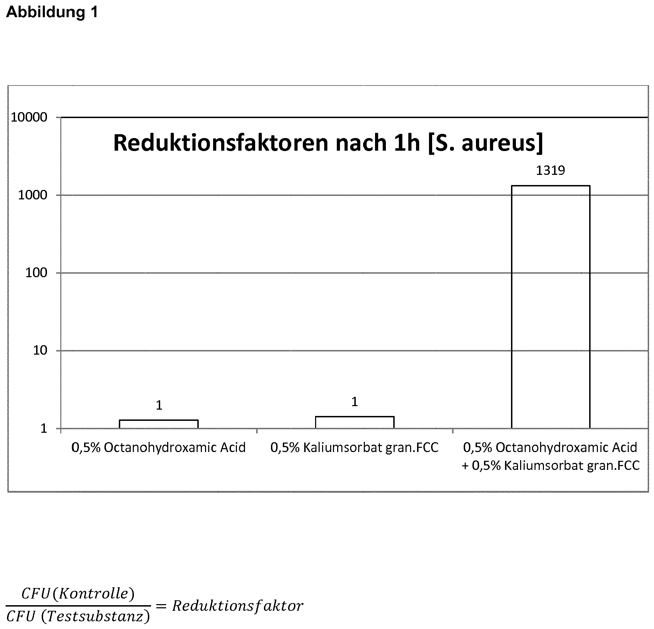

## Patentansprüche

1. Nichttherapeutische Verwendung von Wirkstoffkombinationen, umfassend
a) Sorbinsäure
und
b) eine oder mehrere physiologisch unbedenkliche Hydroxamsäuren zur Bekämpfung oder Prophylaxe von Kopfschuppen.

2. Verwendung nach Anspruch 1, **dadurch gekennzeichnet, daß** die Gewichtsverhältnisse der Hydroxamsäure, insbesondere Octanohydroxamsäure, einerseits zu Sorbinsäure andererseits, aus dem Bereich 50 : 1 bis 1 : 50, vorzugsweise 5 : 1 bis 1 : 5 gewählt werden.

3. Verwendung nach Anspruch 1 oder 2, in Zubereitungen enthaltend 0,001 bis 5,0 Gew.-% Hydroxamsäure enthalten, bevorzugt 0,01 bis 2,0 Gew.-%, bezogen auf das Gesamtgewicht der Zubereitungen.

4. Verwendung nach einem der vorhergehenden Ansprüche, **dadurch gekennzeichnet, daß** die Zubereitungen 0,001 bis 5,0 Gew.-% an Sorbinsäure, enthalten, bevorzugt 0,01 bis 2,0 Gew.-%, jeweils bezogen auf das Gesamtgewicht der Zubereitungen.

5. Verwendung einem der vorhergehenden Ansprüche **dadurch gekennzeichnet daß** die als Hydroxamsäure die Octanohydroxamsäure gewählt wird.

## Claims

1. Non-therapeutic use of active ingredient combinations comprising
a) sorbic acid
and
b) one or more physiologically safe hydroxamic acids
for controlling or prophylaxis of dandruff.

2. Use according to Claim 1, **characterized in that** the ratios by weight of the hydroxamic acid, in particular octanohydroxamic acid on the one hand, to sorbic acid on the other hand, are selected from the range 50:1 to 1:50, preferably 5:1 to 1:5.

3. Use according to Claim 1 or 2, in preparations comprising 0.001 to 5.0% by weight hydroxamic acid, preferably comprise 0.01 to 2.0% by weight, based on the total weight of the preparations.

4. Use according to any of the preceding claims, **characterized in that** the preparations comprise 0.001 to 5.0% by weight of sorbic acid, preferably 0.01 to 2.0% by weight, based in each case on the total weight of the preparations.

5. Use according to any of the preceding claims, **characterized in that** octanohydroxamic acid is selected as hydroxamic acid.

## Revendications

1. Utilisation non thérapeutique de combinaisons d'agents actifs, comprenant :
a) de l'acide sorbique
et
b) un ou plusieurs acides hydroxamiques physiologiquement inoffensifs,
pour lutter contre ou prévenir les pellicules.

2. Utilisation selon la revendication 1, **caractérisée en ce que** le rapport en poids entre l'acide hydroxamique, notamment l'acide octanohydroxamique, d'une part et l'acide sorbique d'autre part est choisi dans la plage allant de 50:1 à 1:50, de préférence de 5:1 à 1:5.

3. Utilisation selon la revendication 1 ou 2, dans des préparations contenant 0,001 à 5,0 % en poids d'acide hydroxamique, de préférence 0,01 à 2,0 % en poids, par rapport au poids total des préparations.

4. Utilisation selon l'une quelconque des revendications précédentes, **caractérisée en ce que** les préparations contiennent 0,001 à 5,0 % en poids d'acide sorbique, de préférence 0,01 à 2,0 % en poids, à chaque fois par rapport au poids total des préparations.

5. Utilisation selon l'une quelconque des revendications précédentes, **caractérisée en ce que** l'acide octanohydroxamique est choisi en tant qu'acide hydroxamique.
